# FASCICULE DE BREVET EUROPEEN

(11) **EP 0 885 232 B1**
(45) Date de publication et mention de la délivrance du brevet: **15.01.2003**
(21) Numéro de dépôt: 97907142.0
(22) Date de dépôt: 27.02.1997
(51) Int. Cl.: C07F 9/60, C07F 9/6558, C07D 215/14, C07D 405/06

(54) **NOUVEAUX DERIVES DE L'ACIDE 1(2H) QUINOLEINE CARBOXYLIQUE, LEUR PROCEDE DE PREPARATION ET LEUR APPLICATION A LA SYNTHESE DE PRODUITS DOUES DE PROPRIETES ANTIBIOTIQUES**
NEUE DERIVATE DER 1(2H) CHINOLIN-CARBONSÄURE, VERFAHREN ZU DEREN HERSTELLUNG UND DEREN VERWENDUNG ZUR HERSTELLUNG VON PRODUKTEN MIT ANTIBIOTISCHEN EIGENSCHAFTEN
NOVEL 1(2H) QUINOLINE CARBOXYLIC ACID DERIVATIVES, METHOD FOR PREPARING SAME, AND USE THEREOF FOR SYNTHESISING SUBSTANCES HAVING ANTIBIOTIC PROPERTIES

(30) Priorité: 28.02.1996 FR 9602473
(43) Date de publication de la demande: 23.12.1998
(73) Titulaire: Aventis Pharma S.A., 92160 Antony (FR)
(72) Inventeur: BONNET, Alain, F-02400 Chateau Thierry (FR); BOUCHET, Raphael, F-93500 Pantin (FR); GUILMARD, Daniel, F-77680 Roissy-en-Brie (FR); MAZURIE, Alain, F-93410 Vaujours (FR)
(86) Numéro de dépôt international: FR9700352
(87) Numéro de publication internationale: WO97031926

(56) Documents cités:
- EP-A- 0 676 409
- TETRAHEDRON LETT. (TELEAY,00404039);82; VOL.23 (16); PP.1709-12, HIROSHIMA UNIV.;DEP. CHEM.; HIROSHIMA; 730; JAPAN (JP), XP000611889 AKIBA K ET AL: "Regioselective synthesis of 4-alkylquinolines from quinoline via 1-ethoxycarbonyl-1,2-dihydroquinoline-2-ph osphonates" cité dans la demande
- CHEMICAL ABSTRACTS, vol. 106, no. 13, 30 Mars 1987 Columbus, Ohio, US; abstract no. 102547, AKIBA K ET AL: "Quinolinephosphonate derivatives" XP002018371 & JP 61 221 192 A (TOYO SODA) 1 Octobre 1986

## Description

La présente invention concerne de nouveaux dérivés de l'acide 1(2H) quinoléine carboxylique, leur procédé de préparation et leur application à la synthèse de produits doués de propriétés antibiotiques.

L'invention a pour objet les composés de formule (I) : 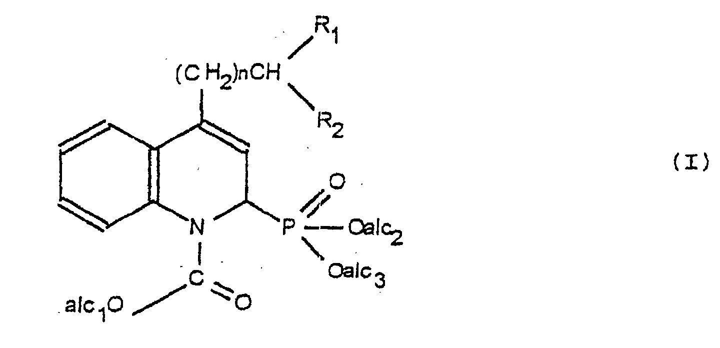 dans lesquels alc₁, alc₂ et alc₃ identiques ou différents l'un de l'autre représentent un radical alkyle renfermant jusqu'à 8 atomes de carbone, n représente un nombre entier pouvant varier de 0 à 8, R₁ et R₂ représentent un radical O-alkyle renfermant jusqu'à 8 atomes de carbone, ou forment ensemble avec l'atome de carbone auquel ils sont liés un acétal cyclique.

Le radical alkyle est de préférence un radical méthyle, éthyle, n-propyle, isopropyle, butyle, isobutyle ou terbutyle.

Lorsque R₁ et R₂ forment avec l'atome de carbone auquel ils sont liés un acétal cyclique, il s'agit de préférence d'un groupement : 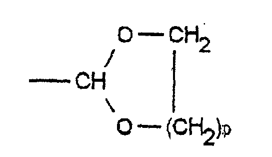 dans lequel p représente le nombre 1, 2, 3 ou 4.

L'invention a tout particulièrement pour objet les composés de formule (I) dans lesquels n représente le nombre 2, ainsi que ceux dans. lesquels alc₁, alc₂ et alc₃ représentent un radical éthyle.

Parmi les composés préférés de l'invention on peut citer les composés de formule (I) dans lesquels R₁ et R₂ forment ensemble avec l'atome de carbone auquel ils sont liés un radical 1,3-dioxolanne : 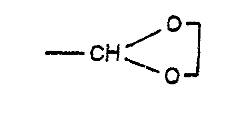 ou 1,3-dioxane

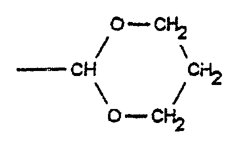

L'invention a tout particulièrement pour objet le composé de formule (I) dont la préparation est donnée ci-après dans la partie expérimentale.

L'invention a également pour objet un procédé de préparation des composés de formule (I), caractérisé en ce que l'on soumet un composé de formule (II) : 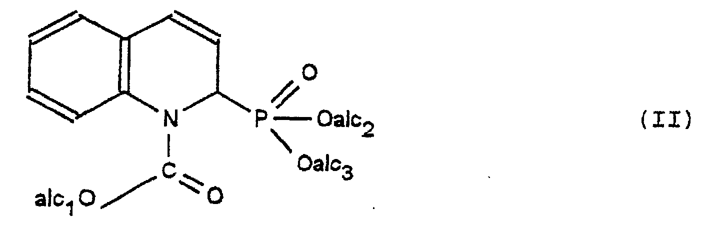 dans laquelle alc₁, alc₂ et alc₃ conservent leur signification précédente, en présence de terbutylate ou de teramylate de sodium ou de potassium à l'action d'un composé de formule (III) : 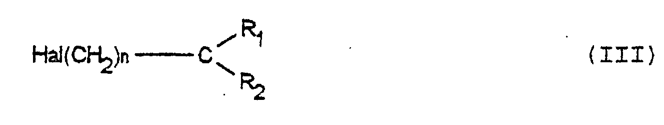 dans laquelle Hal représente un atome d'halogène et R₁, R₂ et n conservent leur signification précédente, pour obtenir le composé de formule (I) correspondant.

Les composés de formule (II) utilisés comme produits de départ du procédé de l'invention peuvent être préparés selon les procédés décrits dans Tetrah. Lett. 23(16), 1709-12 (1982) ou dans la demande de brevet japonais 1221-102.

Dans un mode de réalisation préféré du procédé de l'invention, Hal est un atome de brome ou de chlore.

L'invention a en outre pour objet l'application caractérisée en ce que l'on soumet un composé de formule (I) à l'action d'une base forte pour obtenir le composé de formule (IV) correspondant : 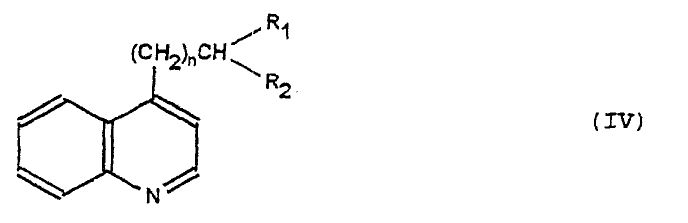 dans laquelle n, R₁ et R₂ conservent leur signification précédente.

L'invention a plus spécialement pour objet l'application caractérisée en ce que la base forte peut être la soude ou de préférence l'éthylate de sodium.

On peut également opérer en présence d'un iodure alcalin comme l'iodure de sodium au sein d'un solvant polaire aprotique, à haute température.

De manière avantageuse, et notamment lorsque R₁ et R₂ forment ensemble avec l'atome de carbone auquel ils sont liés un acétal cyclique, le produit de formule (IV) est purifié sous forme de chlorhydrate mais il peut également être sous forme de l'hydrate d'aldéhyde.

L'invention a plus spécialement pour objet l'application du composé de l'exemple 1 ou 2, à la préparation des produits de formule (IV) décrite dans la partie expérimentale.

Les composés de formule (IV) sont des produits connus d'une façon générale ; ils peuvent être également préparés selon le procédé décrit dans la demande de brevet européen 676409.

Les produits de formule (IV) peuvent être transformés en aldéhydes correspondants : 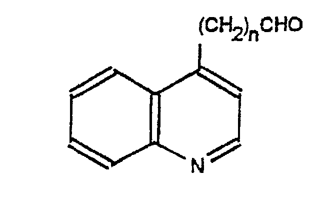 et être ensuite transformés en produits présentant des propriétés antibiotiques selon le procédé décrit dans la demande de brevet précitée.

C'est ainsi qu'à partir du produit de l'application décrite dans la partie expérimentale, on peut préparer la 11,12-didéoxy-3-de((2,6-didéoxy-3-C-méthyl-3-O-méthyl-alpha-L-ribohexopyranosyl) oxy)-6-O-méthyl-3-oxo-12,11-(oxycarbonyl(2-(3-(4-quinoléinyl) propyl) hydrazono))-érythromycine, qui est un produit présentant d'intéressantes propriétés antibiotiques comme il est indiqué dans la demande de brevet européen précitée.

Les exemples suivants illustrent l'invention sans toutefois la limiter.

### EXEMPLE 1 : 2-(diéthoxyphosphinyl)-4-(2-(1,3-dioxolan-2-yl) éthyl)-1(2H)-quinoléine carboxylate d'éthyle

On ajoute à -78°C, 75 ml d'une solution 1,6 M de n-butyllithium dans l'hexane à une solution renfermant 33,933 g de 2-(diéthoxyphosphinyl-1(2H)-quinoléine carboxylate d'éthyle dans 500 ml de tétrahydrofuranne. On ajoute ensuite 18 ml de 2-(2-bromoéthyl)-1,3-dioxolanne. On laisse revenir à la température ambiante, maintient le mélange réactionnel sous agitation pendant 5 heures. On ajoute 300 ml d'eau, agite et laisse décanter. On lave la phase organique à l'eau et la sèche. On filtre et élimine le solvant. On obtient 63,45 g de produit que l'on purifie par chromatographie en éluant à l'acétate d'éthyle. On obtient ainsi le produit recherché. Rf = 0,16. Rdt 62,7 %.
RMN CDCl₃ ppm
0,97 (t), CH₃ de N-COOEt ; 1,18 (t) - 1,33 (t), les CH₃ des P-OEt ; 1,91 (m) (2H), CH-CH₂-CH₂ ; 2,60 (m) (2H), =C-CH₂-CH₂ ; ~ 3,70 à 4,40 (~ 11H), les CH₂ de P-OEt et de COOEt, les O-CH₂ du cétal ; 4,96 (t) (1H), O-CH-CH₂ ; 5,52 (dl) (1H), P-CH-N ; 5,90 (tl), H₃ ; 7,11 (m) (1H), 7,20 à 7,35 (m) (2H).

### APPLICATION 1 : 4-(2-(1,3-dioxolan-2-yl) éthyl-quinoléine

On chauffe au reflux pendant une heure sous azote un mélange renfermant 63,45 g de produit préparé à l'exemple 1, 430 ml d'une solution de soude 2N et 430 ml d'éthanol absolu.

On élimine l'éthanol, extrait à l'éther isopropylique. On lave à l'eau les phases organiques, les sèche et filtre (Rdt = 43 %). On distille le solvant et obtient 11,263 g de produit que l'on purifie par chromatographie sur silice en éluant avec le mélange acétate d'éthyle-cyclohexane 70-30. On obtient le produit recherché. rf = 0,4.
RMN CDCl₃ ppm
2,13 (m), -CH₂- central ; 3,22 (m), =C-CH₂-CH₂- ; 3,86 à 4,11 (m), -O-CH₂-CH₂-O- ; 5,00 (t), O-CH (cétal) ; 7,27 (d,j = 4,5) H₃ ; 8,81 (d,j = 4,5) H₂ ; 7,57 (dt) et 7,71 (dt), H₆ et H₇ ; 8,10 (m, 2H), H₅ et H₈.

### EXEMPLE 2 : 2-(diéthoxyphosphinyl)4-(2-(1,3-dioxan-2-yl) éthyl) 1(2H)-quinoléine carboxylate d'éthyle.

On ajoute à 20,± 2°C, 3,6 g de terbutylate de potassium dans une solution renfermant 9 g de 2-(diéthoxyphosphinyl) 1(2H)-quinoléine carboxylate d'éthyle et 45 ml de diméthylformamide. On maintient sous agitation pendant 30 minutes. On introduit ensuite à -20° ± 2°C, 9,7 g de 2-(2-iodoéthyl) 1,3-dioxane. On ajoute 54 ml d'eau, verse la solution obtenue sur 180 ml d'eau, extrait à l'éther isopropylique, lave à l'eau, sèche, lave de nouveau à l'éther isopropylique. On concentre sous pression réduite pour obtenir 12 g de produit recherché.

### APPICATION 2 : Chlorhydrate de 4-(2-(1,3-dioxan-2-yl)éthyl quinoléine.

On introduit à +20° ± 2°C, 7,5 g d'éthylate de sodium dans une solution renfermant 10 g de produit préparé à l'exemple 1 ou 2 et 50 ml d'éthanol 100. On porte le mélange obtenu au reflux pendant 1 heure. On refroidit à 20° ± 2°C et maintient l'agitation pendant 1 heure et 30 minutes. On distille sous pression réduite et ajoute ensuite à 20° ± 2°C, 50 ml d'eau. On extrait à l'éther isopropylique, lave à l'eau, sèche sur sulfate de sodium, essore et lave à l'éther isopropylique. On concentre et obtient 3,89 g du produit recherché sous forme de base. On met en solution à 20°C 3,76 g du produit obtenu dans 19 ml d'acétate d'éthyle, ajoute en 20 minutes 7,6 ml d'une solution d'acétate d'éthyle à 10% d'acide chlorhydrique, maintient sous agitation pendant 1 heure, filtre, lave le précipité à l'acétate d'éthyle, sèche à 40°C pendant 2 heures et recueille 2,56 g du chlorhydrate attendu.

## Revendications

1. Les composés de formule (I) : 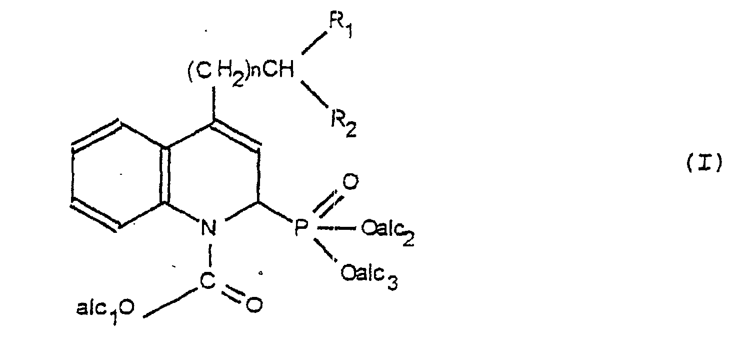 dans lesquels alc₁, alc₂ et alc₃ identiques ou différents l'un de l'autre représentent un radical alkyle renfermant jusqu'à 8 atomes de carbone, n représente un nombre entier pouvant varier de 0 à 8, R₁ et R₂ représentent un radical O-alkyle renfermant jusqu'à 8 atomes de carbone, ou forment ensemble avec l'atome de carbone auquel ils sont liés un acétal cyclique.

2. Les composés de formule (I) tels que définis à la revendication 1, dans lesquels n représente le nombre 2.

3. Les composés de formule (I) tels que définis à la revendication 1 ou 2, dans lesquels alc₁, alc₂ et alc₃ représentent un radical éthyle.

4. Les composés de formule (I) tels que définis à l'une quelconque des revendication 1 à 3, dans lesquels R₁ et R₂ forment ensemble avec l'atome de carbone auquel ils sont liés un radical 1,3-dioxolanne : 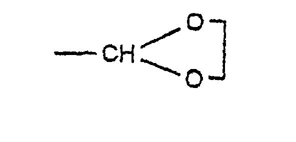 ou un radical 1,3-dioxane

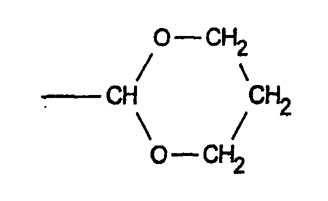

5. Les composés de formule (I) définis à la revendication 1, dont les noms suivent :
- 2-(diéthoxyphosphinyl)-4-(2-(1,3-dioxolan-2-yl) éthyl)-1(2H)-quinoléinecarboxylate d'éthyle,
- 2-(diéthoxyphospninyl) 4- (2-(1,3-dioxan-2-yl) éthyl) 1(2H)-quinoléine carboxylate d'éthyle.

6. Procédé de préparation des composés de formule (I) tels que définis à l'une quelconque des revendications 1 à 5, **caractérisé en ce que** l'on soumet un composé de formule (II) : 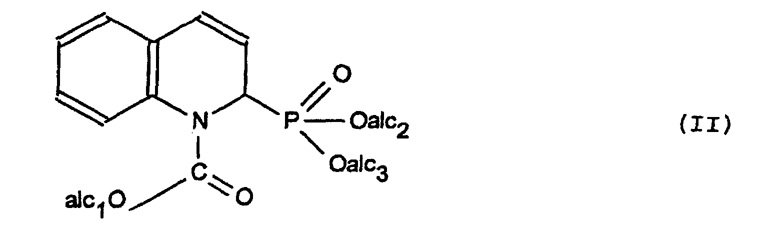 dans laquelle alc₁, alc₂ et alc₃ conservent leur signification précédente, en présence de terbutylate ou de teramylate de sodium ou de potassium, à l'action d'un composé de formule (III) : 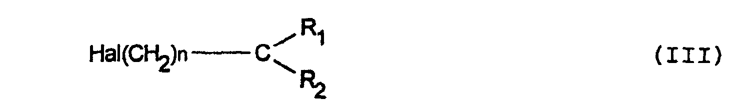 dans laquelle Hal représente un atome d'halogène et R₁, R₂ et n conservent leur signification précédente, pour obtenir le composé de formule (I) correspondant.

7. Utilisation des composés de formule (I) tels que définis à l'une quelconque des revendications 1 à 5, **caractérisée en ce que** l'on soumet un composé de formule (I) à l'action d'une base forte pour obtenir le composé de formule (IV) correspondant : 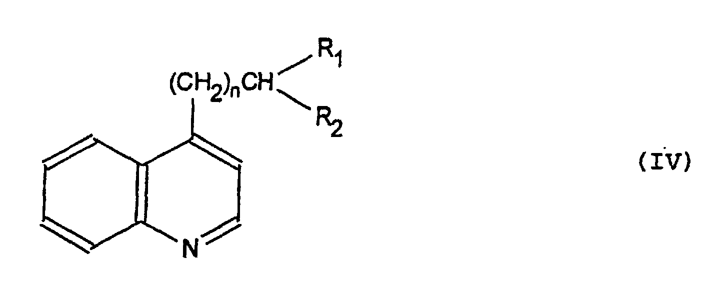 dans laquelle n, R₁ et R₂ conservent leur signification précédente.

8. Utilisation selon la revendication 7, **caractérisée en ce que** la base forte est l'éthylate de sodium.

9. Utilisation selon la revendication 7 ou 8, **caractérisée en ce que** le produit de formule (I) utilisé est le 2-(diéthoxyphosphinyl) 4-(2-(1,3-dioxan-2-yl) éthyl) 1(2H)-quinoléine carboxylate d'éthyle selon la revendication 5 et le produit de formule (IV) préparé est la 4-(2-(1,3-dioxolan-2-yl) éthyl)-quinoléine.

10. Utilisation selon la revendication 7 ou 8, **caractérisée en ce que** le produit de formule (I) utilisé est le 2-(diéthoxyphosphinyl) 4-(2-(1,3-dioxan-2-yl) éthyl) 1(2H)-quinoléine carboxylate d'éthyle selon la revendication 5 et le produit de formule (IV) préparé est la 4-(2-(1,3-dioxan-2-yl) éthyl) quinoléine.

## Claims

1. The compounds of formula (I): 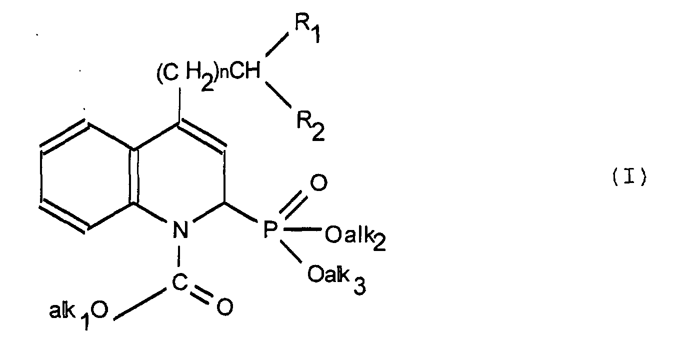 in which alk₁, alk₂ and alk₃ identical to or different from one another represent an alkyl radical containing up to 8 carbon atoms, n represents an integer which can vary from 0 to 8, R₁ and R₂ represent an O-alkyl radical containing up to 8 carbon atoms, or together with the carbon atom to which they are linked form a cyclic acetal.

2. The compounds of formula (I) as defined in claim 1, in which n represents the number 2.

3. The compounds of formula (I) as defined in claim 1 or 2, in which alk₁, alk₂ and alk₃ represent an ethyl radical.

4. The compounds of formula (I) as defined in any one of claims 1 to 3, in which R₁ and R₂ together with the carbon atom to which they are linked form a 1,3-dioxolane radical: 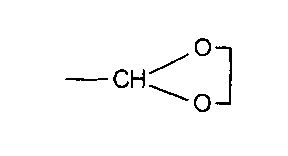 or a 1,3-dioxane radical

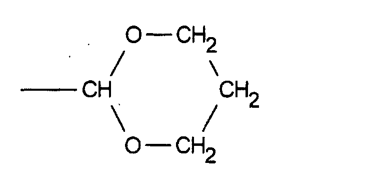

5. The compounds of formula (I) defined in claim 1, the names of which follow:
- ethyl 2-(diethoxyphosphinyl)-4-(2-(1,3-dioxolan-2-yl) ethyl)-1(2H)-quinoline carboxylate,
- ethyl 2-(diethoxyphosphinyl) 4-(2-(1,3-dioxan-2-yl) ethyl) 1(2H)-quinoline carboxylate.

6. Process for the preparation of the compounds of formula (I) as defined in any one of claims 1 to 5, **characterized in that** a compound of formula (II): 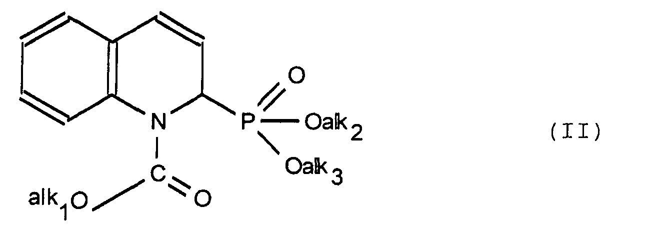 in which alk₁, alk₂ and alk₃ retain their previous meaning, is subjected in the presence of sodium or potassium terbutylate or teramylate, to the action of a compound of formula (III): 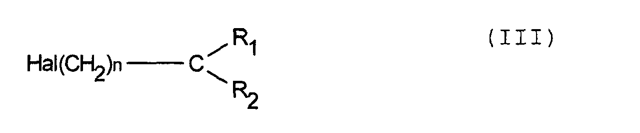 in which Hal represents a halogen atom and R₁, R₂ and n retain their previous meaning, in order to obtain the corresponding compound of formula (I).

7. Use of the compounds of formula (I) as defined in any one of claims 1 to 5, **characterized in that** a compound of formula (I) is subjected to the action of a strong base in order to obtain the corresponding compound of formula (IV): 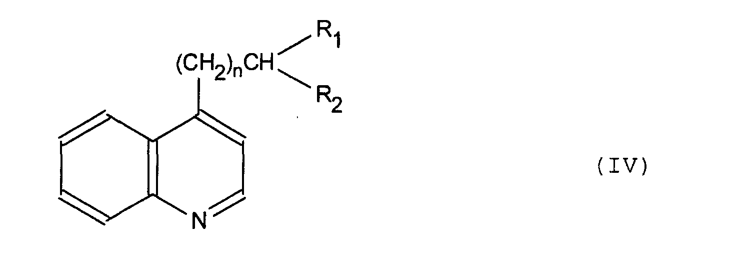 in which n, R₁ and R₂ retain their previous meaning.

8. Use according to claim 7, **characterized in that** the strong base is sodium ethylate.

9. Use according to claim 7 or 8, **characterized in that** the product of formula (I) used is ethyl 2-(diethoxyphosphinyl) 4-(2-(1,3-dioxan-2-yl) ethyl) 1(2H)-quinoline carboxylate according to claim 5 and the product of formula (IV) prepared is 4-(2-(1,3-dioxolan-2-yl) ethyl)-quinoline.

10. Use according to claim 7 or 8, **characterized in that** the product of formula (I) used is ethyl 2-(diethoxyphosphinyl) 4-(2-(1,3-dioxan-2-yl) ethyl) 1(2H)-quinoline carboxylate according to claim 5 and the product of formula (IV) prepared is 4-(2-(1,3-dioxan-2-yl) ethyl) quinoline.

## Patentansprüche

1. Verbindungen der Formel (I) 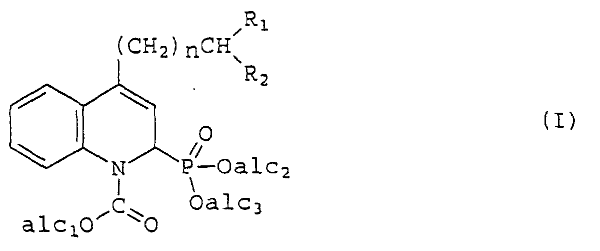 in der alc₁, alc₂ und alc₃, untereinander gleich oder verschieden, einen Rest Alkyl mit bis zu 8 Kohlenstoffatomen darstellen, n eine ganze Zahl von 0 bis 8 ist, R₁ und R₂ einen Rest O-Alkyl mit bis zu 8 Kohlenstoffatomen bedeuten oder zusammen mit dem Kohlenstoffatom, an das sie gebunden sind, ein cyclisches Acetal bilden.

2. Verbindungen der Formel (I) wie in Anspruch 1 definiert, worin n die Zahl 2 darstellt.

3. Verbindungen der Formel (I) wie in Anspruch 1 oder 2 definiert, worin alc₁, alc₂ und alc₃ einen Rest Ethyl darstellen.

4. Verbindungen der Formel (I) wie in irgendeinem der Ansprüche 1 bis 3 definiert, worin R₁ und R₂ zusammen mit dem Kohlenstoffatom, an das sie gebunden sind, einen Rest 1,3-Dioxolan 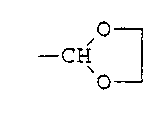 oder einen Rest 1,3-Dioxan 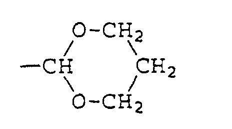 bilden.

5. Verbindungen der Formel (I) wie in Anspruch 1 definiert, mit den folgenden Namen:
- 2-(Diethoxyphosphinyl)-4-[2-(1,3-dioxolan-2-yl)-ethyl]-1(2H)-chinolincarbonsäure-ethylester
- 2-(Diethoxyphosphinyl)-4-[2-(1,3-dioxan-2-yl)-ethyl]-1(2H)-chinolincarbonsäure-ethylester.

6. Verfahren zur Herstellung der Verbindungen der Formel (I) wie in irgendeinem der Ansprüche 1 bis 5 definiert, **dadurch gekennzeichnet, daß** man eine Verbindung der Formel (II) 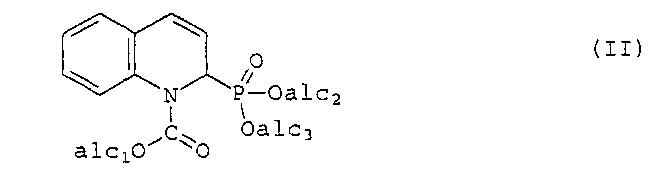 in der alc₁, alc₂ und alc₃ ihre vorstehend genannte Bedeutung beibehalten, in Anwesenheit von tert.-Butylat oder tert.-Amylat von Natrium oder Kalium der Einwirkung einer Verbindung der Formel (III) 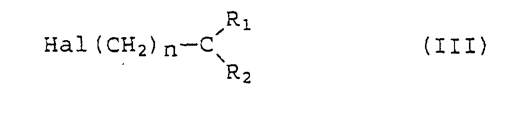 unterzieht, in der Hal ein Halogenatom ist und R₁, R₂ und n ihre vorstehend genannte Bedeutung beibehalten, um die entsprechende Verbindung der Formel (I) zu erhalten.

7. Verwendung der Verbindungen der Formel (I) wie in irgendeinem der Ansprüche 1 bis 5 definiert, **dadurch gekennzeichnet, daß** man eine Verbindung der Formel (I) der Einwirkung einer starken Base unterzieht, um die entsprechende Verbindung der Formel (IV) 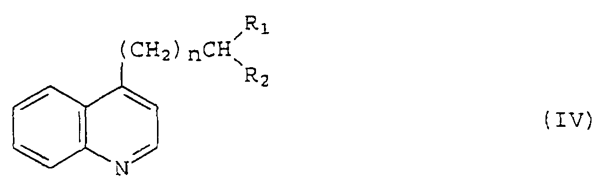 zu erhalten, in der n, R₁ und R₂ ihre vorstehend genannte Bedeutung beibehalten.

8. Verwendung nach Anspruch 7, **dadurch gekennzeichnet, daß** die starke Base Natriumethylat ist.

9. Verwendung nach Anspruch 7 oder 8, **dadurch gekennzeichnet, daß** das verwendete Produkt der Formel (I) 2-(Diethoxyphosphinyl)-4-[2-(1,3-dioxolan-2-yl)-ethyl]-1(2H)-chinolincarbonsäure-ethylester gemäß Anspruch 5 ist und das hergestellte Produkt der Formel (IV) 4-[2-(1,3-Dioxolan-2-yl)-ethyl]-1(2H)-chinolincarbonsäure-ethylester ist.

10. Verwendung nach Anspruch 7 oder 8, **dadurch gekennzeichnet, daß** das verwendete Produkt der Formel (I) 2-(Diethoxyphosphinyl)-4-[2-(1,3-dioxan-2-yl)-ethyl]-1(2H)-chinolincarbonsäure-ethylester gemäß Anspruch 5 ist und das hergestellte Produkt der Formel (IV) 4-[2-(1,3-Dioxan-2-yl)-ethyl]-1(2H)-chinolincarbonsäureethylester ist.
